# EUROPEAN PATENT APPLICATION

(11) **EP 4 840 774 A1**
(43) Date of publication of application: **18.11.2026**
(21) Application number: 26176382.5
(22) Date of filing: 05.05.2026
(51) Int. Cl.: B65B 9/20, B65B 55/10, B65B 55/24, B65B 59/04, A61L 2/208

(54) **A METHOD FOR DISMOUNTING A HYDROGEN PEROXIDE REMOVAL STATION**

(30) Priority: 12.05.2025 IT 202500010662
(71) Applicant: Tetra Laval Holdings & Finance S.A., 1009 Pully (CH)
(72) Inventor: ALGERI, Pierpaolo, 41123 Modena (IT)
(74) Representative: Tetra Pak Patent Attorneys

(57) **Abstract**

A method (2300) for dismounting a hydrogen peroxide removal station (108) is disclosed. The hydrogen peroxide removal station (108) comprises a calendar roller assembly (CRA) (200) comprising a CRA shaft (400), a squeegee roller assembly (SRA) (204) comprising a SRA shaft (1400), a drying chamber (208), a CRA attachment member (202) for holding the CRA (200) in position within the drying chamber (208), and a SRA attachment member (206) for holding the SRA (204) in position within the drying chamber (208). The method comprises removing (2302) a CRA locking pin (402) such that the CRA attachment member (202) is disengaged from the CRA (200), sliding (2304) the CRA attachment member (202) along a CRA pivot axis (CRA PA) away from the CRA shaft (400), releasing (2306) a second end of the CRA (200) by rotating the CRA shaft (400) around the CRA PA, removing (2308) the CRA (200) from the drying chamber (208), removing (2310) a SRA locking pin (1402) such that the SRA attachment member (206) is disengaged from a SRA shaft (1400), sliding (2312) the SRA attachment member (206) along the SRA RA away from the SRA shaft (1400), releasing (2314) a second end of the SRA (204) by rotating the SRA shaft (1400) around the SRA RA, and removing (2316) the SRA (204) from the drying chamber (208).

## Description

### Technical Field

The invention relates to packaging technology. More particularly, it relates to methods for dismounting a hydrogen peroxide removal station placed downstream a hydrogen peroxide bath arranged for sterilizing a web of packaging material.

### Background Art

Today it is well known to use hydrogen peroxide in packaging machines, sometimes also referred to as filling machines, for sterilizing packaging material. A known packaging machine is disclosed in patent document JPH10114211A. By way of example, in roll-fed packaging machines, it is a common approach to feed a web of packaging material into a hydrogen peroxide bath to ensure that bacteria, spores and other unwanted microorganisms are killed off before the web is formed into a tube and filled with food product.

To remove residual hydrogen peroxide, it is known to have a hydrogen peroxide removal station placed downstream the hydrogen peroxide bath. Such station may be arranged with two rollers. By having the web fed between these two rollers, the residual hydrogen peroxide on the web can be removed. It is also known to use so-called air knives for removing the residual hydrogen peroxide.

In addition, hot sterile air may be used in the hydrogen peroxide removal station. By having the web exposed to the sterile air, the residual hydrogen peroxide is evaporated. Thus, by combining the use of rollers and the use of hot sterile air, the residual hydrogen peroxide can be removed efficiently.

Even though the hydrogen peroxide removal stations used today ensure that the residual hydrogen peroxide is removed, there is room for improvement in the design of the station to enhance ease of maintenance and serviceability.

### Summary

It is an object of the invention to at least partly overcome one or more of the above-identified limitations of the prior art. In particular, it is an object to provide a hydrogen peroxide removal station that is arranged such that a calendar roller assembly and a squeegee roller assembly can easily be removed by a service technician, thereby reducing the down time of the filling machine. In addition, it is also an object to have the hydrogen peroxide removal station arranged such that a risk that there is contact between surfaces of the rollers and other parts of the station during the dismounting process is lowered. As an effect, the risk that the rollers need to be replaced due to that the surfaces are scratched or in other way damaged, can be reduced.

According to a first aspect it is provided a method for dismounting a hydrogen peroxide removal station arranged for removing residual hydrogen peroxide from a web of packaging material downstream a hydrogen peroxide bath in a filling machine, wherein the hydrogen peroxide removal station comprises a calendar roller assembly CRA comprising a CRA shaft, a squeegee roller assembly SRA comprising a SRA shaft, a drying chamber, a CRA attachment member for holding the CRA in position within the drying chamber, and a SRA attachment member for holding the SRA in position within the drying chamber, said method comprising
removing a CRA locking pin such that the CRA attachment member is disengaged from the CRA,
sliding the CRA attachment member along a CRA pivot axis away from the CRA,
releasing a second end of the CRA by rotating the CRA around the CRA pivot axis,
removing the CRA from the drying chamber,
removing a SRA locking pin such that the SRA attachment member is disengaged from a SRA shaft,
sliding the SRA attachment member along the SRA RA away from the SRA shaft,
releasing a second end of the SRA by rotating the SRA around the SRA RA, and
removing the SRA from the drying chamber.

An advantage with the approach suggested above is that the risk that the CRA and SRA are not securely fastened during operation is low due to the use of the CRA locking pin and the SRA locking pin, respectively. A further advantage is that the CRA as well as the SRA can be released from an outside of the drying chamber. Thus, in contrast to a solution in which the service technician needs to unscrew a bolt with a wrench inside the drying chamber, the service technician may, with the approach suggested above, release the CRA as well as the SRA from the outside, which provides for better working ergonomic. Further, by avoiding the need to unscrew the CRA and SRA from the inside, a risk of scratching surfaces of the CRA and the SRA can be reduced.

During operation, hot air may be introduced into the hydrogen peroxide removal station. The hot air may, in combination with the hydrogen peroxide, result in that deposits are formed on the CRA and the SRA.

The hot air removes the residual hydrogen peroxide by evaporation, but at the risk of forming the deposits on the rollers. Thus, when having the hydrogen peroxide removed by a combination of mechanical removal, e.g. by using the rollers, and heat removal, by using the hot air, a service need for the CRA and the SRA may be increased. Therefore, having an efficient and secure way of providing service is of particular relevance if adding the hot air.

The drying chamber may comprise a first side section and a second side section, wherein the CRA attachment member and the SRA attachment member may be arranged in a first and a second opening in the first side section, wherein the CRA locking pin and the SRA locking pin may be provided inside the drying chamber, and a CRA and a SRA grip may be provided outside the drying chamber such that the steps of sliding the CRA attachment member and sliding the SRA attachment member can be achieved by pulling the grips away from the drying chamber.

A CRA lifting knob may be provided, and the method may further comprise lifting the CRA lifting knob such that the CRA attachment member is allowed to slide along the CRA pivot axis.

By having the CRA lifting knob, the service technician may pull the lifting knob upwards by one hand at the same time as the CRA attachment member is pulled away from the drying chamber by the other hand. In this way, a risk of unintentionally releasing the CRA can be held low at the same time as the CRA can be removed efficiently when this is to be serviced.

The CRA attachment member may be provided with an operational state slot and a service state slot such that by lifting the CRA lifting knob and sliding the CRA attachment member along the CRA pivot axis while the CRA lifting knob is lifted, the CRA lifting knob can be moved between the operational state slot and the service state slot.

The CRA lifting knob may be spring-loaded.

The CRA may further comprise a first CRA side section, a second CRA side section and a CRA bar, wherein the CRA shaft may be connected to a first end of the first and second CRA side section and the CRA bar may be connected to a second end of the first and second CRA side section.

By having the CRA arranged in this manner comes with two advantages. The CRA may be pivotally arranged such that this can be pressed against the SRA during operation. Further, the CRA bar may be used as a grip for the service technician during removal of the CRA such that the risk of scratching the rollers of the CRA can be held low.

In an operational state, the CRA attachment member may be engaging with the first CRA side section and a fixed CRA attachment member may be engaging with the second CRA side section, wherein the fixed CRA attachment member may be fed through a hole in the second CRA side section, and the CRA locking pin may be attached to the fixed CRA attachment member such that movement of the CRA shaft assembly along the CRA pivot axis is prevented. As an effect, by hindering movement along the CRA pivot axis, movement along the CRA rotational axis is also prevented.

The CRA locking pin may comprise an R-spring.

The CRA locking pin may further comprise a ring having a diameter of 2-4 cm, wherein the ring is attached to the R-spring.

By having the ring, it is made possible to insert a finger into the ring such that the CRA locking pin can be held securely during de-assembly, thereby reducing the risk that the CRA locking pin is dropped and end up in e.g. the hydrogen peroxide bath.

According to a second aspect it is provided a hydrogen peroxide removal station comprising a calendar roller assembly CRA comprising a CRA shaft, a squeegee roller assembly SRA comprising a SRA shaft, a drying chamber, a CRA attachment member for holding the CRA in position within the drying chamber, and a SRA attachment member for holding the SRA in position within the drying chamber, a CRA locking pin arranged to be placed in a hole in the CRA such that the CRA is prevented from moving, wherein the CRA attachment member is arranged to be slid along a CRA pivot axis away from the CRA after the CRA locking pin is removed, a SRA locking pin arranged to be placed in a hole in the SRA such that the SRA is prevented from moving, wherein the SRA attachment member is arranged to be slid along a SRA rotational axis SRA RA away from the SRA shaft after the SRA locking pin is removed.

The hole in which the CRA locking pin is placed may be arranged such that this extend over both the second CRA side section and the fixed CRA attachment member. This, combined with the fact that the fixed CRA attachment member is secured, ensures that the CRA is prevented from moving about the CRA pivot axis.

The same features and advantages as presented above with respect to the first aspect also apply to this aspect.

The hydrogen peroxide removal station may further comprise a hot air nozzle arranged, during operation, to feed in hot air into the hydrogen peroxide removal station.

The drying chamber may comprise a first side section and a second side section, wherein the CRA attachment member and the SRA attachment member are arranged in a first and a second opening in the first side section, wherein the CRA locking pin and the SRA locking pin is provided inside the drying chamber, and a CRA and a SRA grip are provided outside the drying chamber.

According to a third aspect it is provided a filling machine comprising a hydrogen peroxide bath and the hydrogen peroxide removal station according to the second aspect.

According to a fourth aspect, a kit of parts is provided for upgrading an existing hydrogen peroxide removal station comprising a drying chamber into the hydrogen peroxide removal station according to the second aspect. The kit of parts may comprise a calendar roller assembly CRA comprising a CRA shaft, a squeegee roller assembly SRA comprising a SRA shaft, a CRA attachment member for holding the CRA in position within the drying chamber, and a SRA attachment member for holding the SRA in position within the drying chamber, a CRA locking pin arranged to be placed in a hole in the CRA such that the CRA attachment member is prevented from moving, wherein the CRA attachment member is arranged to be slid along a CRA pivot axis away from the CRA after the CRA locking pin is removed, a SRA locking pin arranged to be placed in a hole in the SRA such that the SRA is prevented from moving, wherein the SRA attachment member is arranged to be slid along a SRA rotational axis SRA RA away from the SRA shaft after the SRA locking pin is removed.

Still other objectives, features, aspects and advantages of the invention will appear from the following detailed description as well as from the drawings.

### Brief Description of the Drawings

Embodiments of the invention will now be described, by way of example, with reference to the accompanying schematic drawings, in which
Fig. 1 illustrates a general example of roll-fed packaging machine.
Fig. 2 illustrates a hydrogen peroxide removal station.
Fig. 3 illustrates the hydrogen peroxide removal station in further detail.
Fig. 4 illustrates a cross-sectional view of the calendar roller assembly CRA.
Fig. 5 illustrates a calendar roller assembly locking pin.
Fig. 6 illustrates a perspective view of the CRA.
Fig. 7 illustrates a detailed cross-sectional view of a CRA attachment member.
Fig. 8 to 13 illustrates, by way of example and schematically, different steps in a process of removing the calendar roller assembly.
Fig. 14 illustrates a cross-sectional view of a squeegee roller assembly SRA.
Fig. 15 illustrates a SRA locking pin.
Fig. 16 illustrates a detailed cross-sectional view of a SRA attachment member.
Fig. 17 to 22 illustrates, by way of example, different steps in a process of removing the squeegee roller assembly.
Fig. 23 is a flowchart illustrating a method for dismounting a hydrogen peroxide removal station.

### Detailed Description

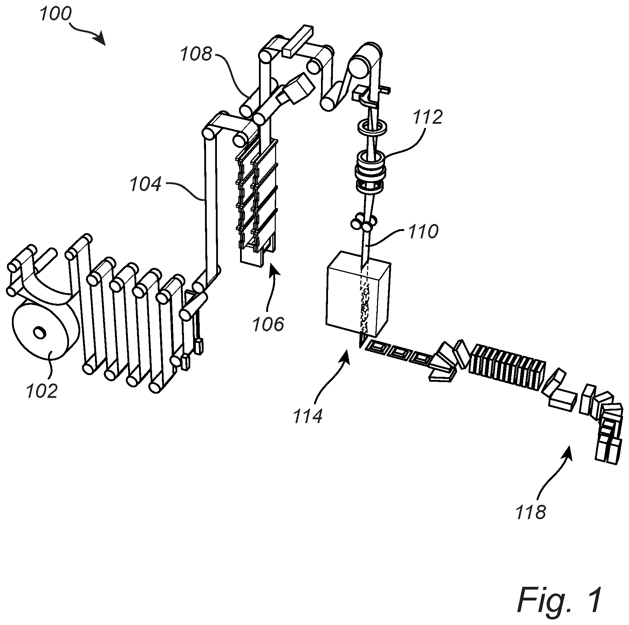

Fig. 1 generally illustrates by way of example a filling machine 100, more particularly a roll-fed filling machine producing carton packages. As illustrated, the filling machine 100, also referred to as packaging machine, is arranged to produce packages 118 filled with food product from a reel 102 of packaging material. From the reel, a web 104 of packaging material is fed into a hydrogen peroxide bath 106. Downstream the bath, a hydrogen peroxide removal station 108 is provided. After the web has been sterilized and the residual hydrogen peroxide is removed, the web is formed into a tube 110 by using a longitudinal sealing station 112. Food product may be filled into the tube 110 from above, thereby providing for that the packages can be produced continuously. In a lower end of the tube, a transversal sealing station 114 is provided. The transversal sealing station 114 may be a chain-based transversal sealing station comprising a first and a second chain. By having this arrangement, it is made possible to produced packages at high speeds, e.g. 30 000 packages per hour or more.

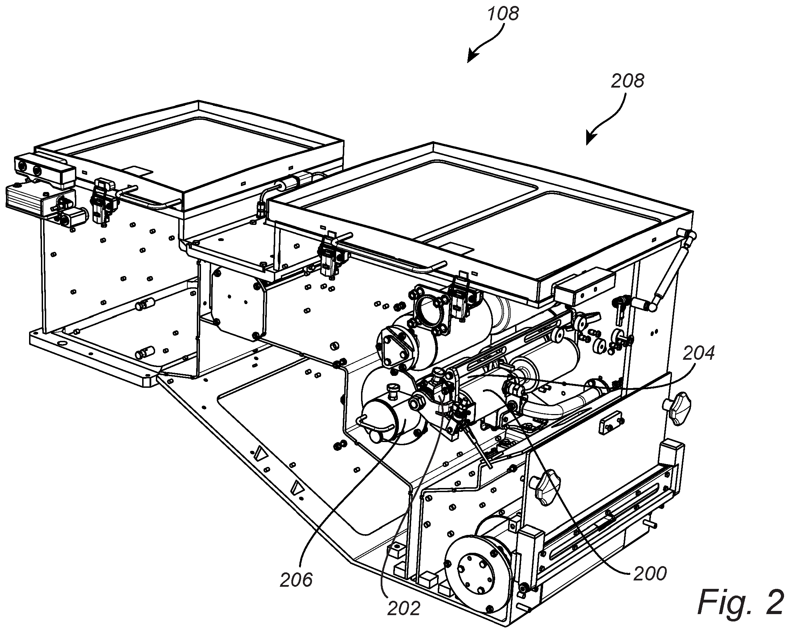

The hydrogen peroxide removal station 108, generally illustrated in fig. 1, may be embodied as illustrated in detail in fig. 2. The hydrogen peroxide removal station 108, illustrated by way of example, can comprise a calendar roller assembly CRA 200 and a squeegee roller assembly SRA 204. By having the web fed between these two assemblies, the residual hydrogen peroxide can be removed. The CRA 200 can be attached to a drying chamber 208 via a CRA attachment member 202, and the SRA 204 may be attached to the drying chamber 208 via a SRA attachment member 206. To provide for that efficient removal is provided, the SRA can be fixedly arranged and the CRA can be pivotally mounted. The roller or rollers used in the CRA and the SRA may be made of rubber or may be provided with an outer layer of rubber, or other non-rigid material, such that the residual hydrogen peroxide can be removed without damaging the packaging material. This is especially relevant if the packaging material is provided with crease lines, that is, weakening lines, for facilitating folding of the packaging material into the packages. By having the rubber, it is possible to provide flexibility such that the rollers can adapt to irregularities of the packaging material.

The drying chamber 208 should be interpreted broadly as a mechanical framework or support system that holds and aligns the CRA and the SRA and in which hydrogen peroxide is removed. The CRA 200 should in this context be understood as an assembly comprising one or more rollers arranged to interact with the SRA, which also should be understood to be an assembly comprising one or more rollers, such that the residual hydrogen peroxide can be removed.

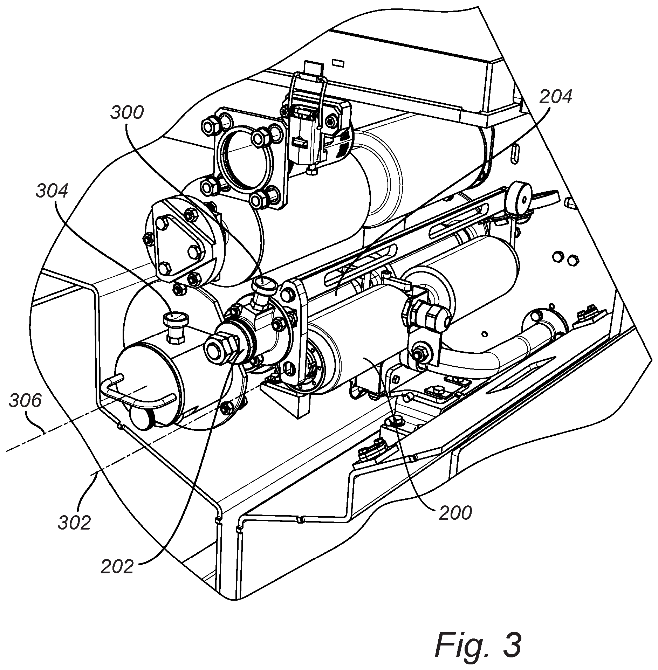

Fig. 3 illustrates the hydrogen peroxide removal station 108 in further detail. As illustrated, in connection to the CRA attachment member 202, a CRA lifting knob 300 may be provided. By lifting the CRA lifting knob 300, the CRA attachment member 202 may be slid away from the drying chamber and also the CRA 200 such the CRA attachment member 202 is axially disjoined from the CRA 200. After having the attachment member disjoined, an opposite end of the CRA 200, herein also referred to as a second end, may be released from the drying chamber 208. As illustrated, the CRA 200 may be arranged such that the CRA rotational axis, CRA RA 302, is parallel and non-coincident with a longitudinal extension of the CRA attachment member 202. As illustrated, the SRA 204 may also be provided a lifting knob in the form of a SRA lifting knob 304. However, unlike the CRA 200, a longitudinal extension of the SRA attachment member 206 may be parallel and coincide with a SRA rotational axis RA, as illustrated.

As illustrated, the CRA 200 may be pivotally mounted such that the rollers of the CRA 200 may be pushed towards the rollers of the SRA 204. The pivot axis can coincide with the longitudinal extension of the CRA attachment member 202.

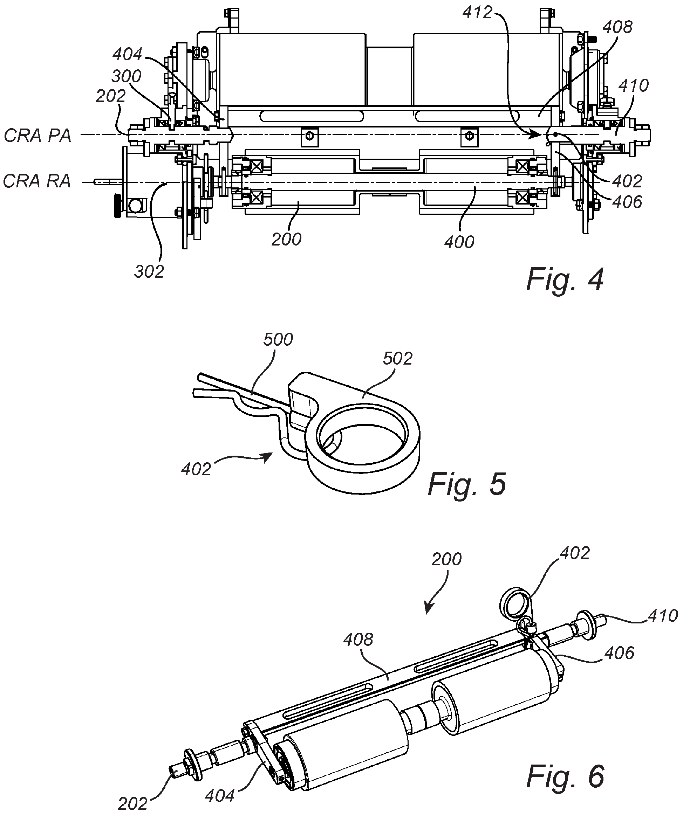

Fig. 4 is a cross-sectional view of the CRA 200 and the CRA attachment member 202. As illustrated, a CRA shaft 400, onto which the roller or rollers of the CRA 200 are arranged can be provided. The CRA shaft 400 can be connected to a first and a second side section 404, 406, respectively. The first side section 404 may be arranged to interact with the CRA attachment member 202, and the second side section 406 may be arranged to interact with a fixed CRA attachment member 410. As the CRA attachment member 202, the fixed CRA attachment member 410 may be attached to the drying chamber 208, but unlike the CRA attachment member 202, the fixed CRA attachment member 410 may not be arranged to be slid between an operational state and a service state. As illustrated, the fixed CRA attachment member 410 may be provided with a hole 412. A locking pin 402 may be placed in the hole 412, thereby locking the CRA 200 in position. To provide stability to the CRA 200 and also to provide a grip for the service technician, the first and second CRA side sections 404, 406 may in their ends facing away from the ends in which the CRA shaft 400 are attached, be attached to a CRA bar 408.

During service, when the CRA 200 is to be removed, the locking pin 402 may be pulled out from the hole 412. Once the locking pin 402 is pulled out, the lifting knob 300 may be lifted, and thereafter the CRA attachment member 202 may be slid away from the drying chamber such that the first side section 404 is de-attached. Thereafter, while holding the CRA bar 408, the service technician can de-attach the second CRA side section 406 from the drying chamber 208, e.g. by rotating the CRA 200.

As illustrated, the CRA rotational axis CRA RA 302 may be parallel with the CRA pivot axis CRA PA.

Fig. 5 illustrates the locking pin 402 in further detail. As illustrated, the locking pin 402 may comprise an R-spring 500. To facilitate for the service technician, the locking pin may further comprise a ring arranged such that the locking pin 402 can be securely held by the service technician during disassembly. For instance, the service technician may place a finger inside the ring and by doing so reduce the risk of losing the locking pin during service.

The size, shape and dimensions of the ring may be chosen such that it can be prevented that this falls into the bath 106 during de-assembly. The bath may be provided with a slot for receiving the web of packaging material. The ring's shape, size and/or dimensions may be adapted such that this cannot pass this slot.

Fig. 6 illustrates the CRA 200 in isolation with part of the CRA attachment member 202 in place and also part of the fixed CRA attachment member 410 in place.

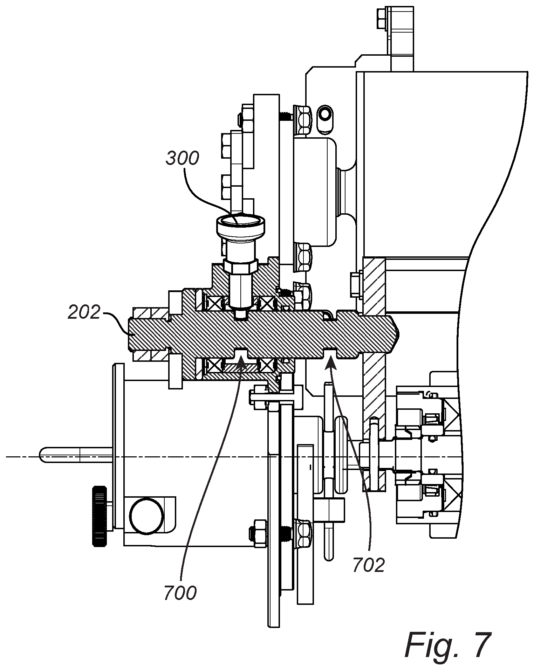

Fig. 7 illustrates the CRA attachment member 202 in further detail. As illustrated, two slots may be provided on this member. During operation, i.e. when the CRA 200 are to be held in place, the CRA lifting knob 300 may interact with an operational state 700, as illustrated. During service, i.e. when the CRA 200 is to be removed, the CRA attachment member 202 slides away from the drying chamber 208, and to hold the CRA attachment member 202 in position, the lifting knob 300 interacts with a service state slot 702.

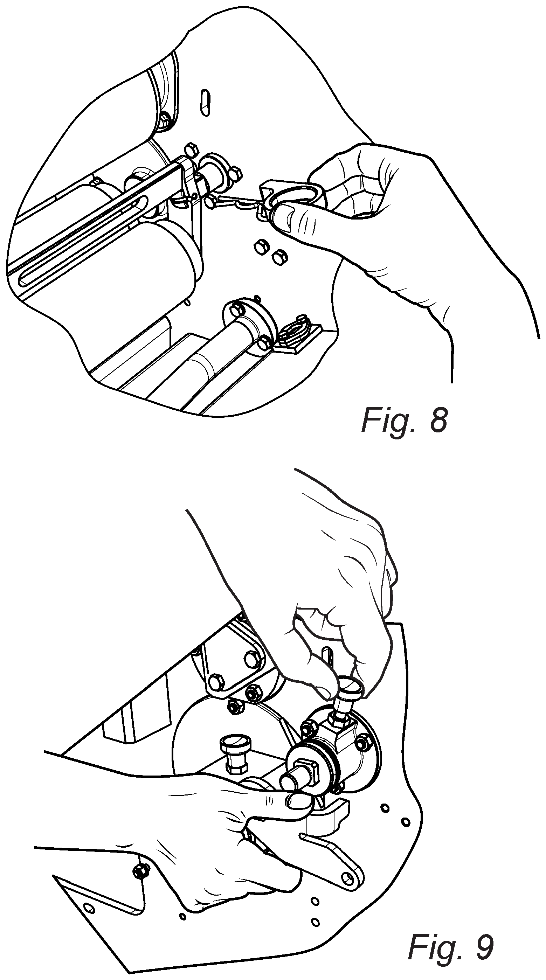

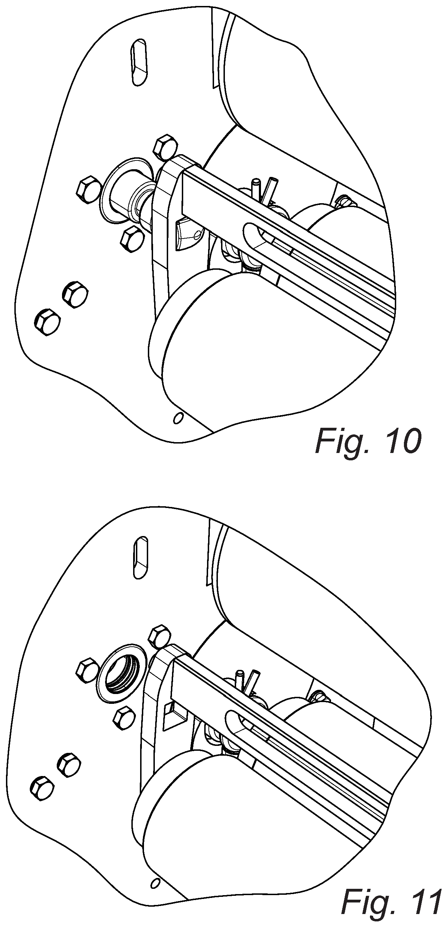

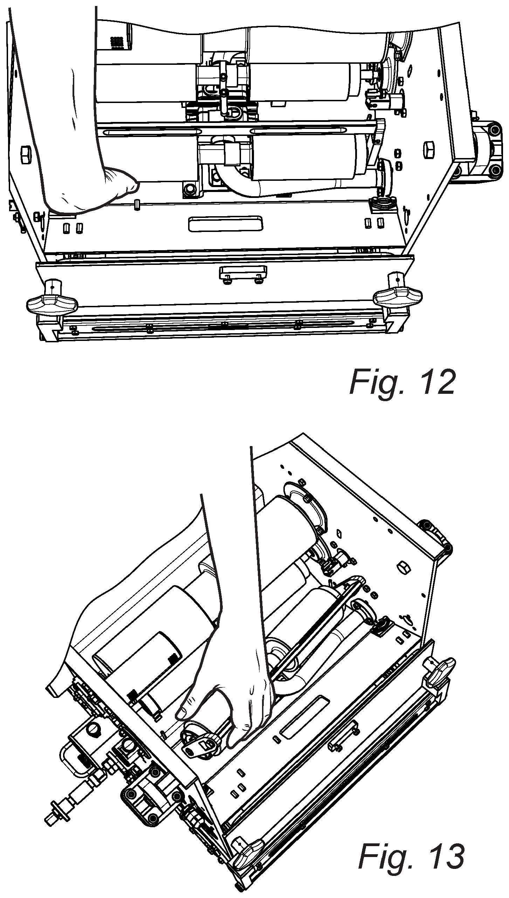

Fig. 8 to 13 illustrate by way of example how the CRA 200 can be removed. In fig. 8, the locking pin is removed by the service technician. In fig. 9, the lifting knob 300 is pulled upwards at the same time as the CRA attachment member 202 is pulled away from the drying chamber. Fig. 10 illustrates the CRA 200 in the operational state, i.e. the CRA attachment member 202 is interacting with the first CRA side section 404 of the CRA 200. Fig. 11 illustrates the CRA 200 in the service state, i.e. the CRA attachment member 202 is disjoined from the first CRA side section 404. Fig. 12 illustrates how the service technician can grab the CRA 200 in the service state and initiate a removal of the CRA 200. Fig. 13 illustrates how the service technician can disconnect the CRA 200 from the fixed CRA attachment member 410 by rotating the CRA.

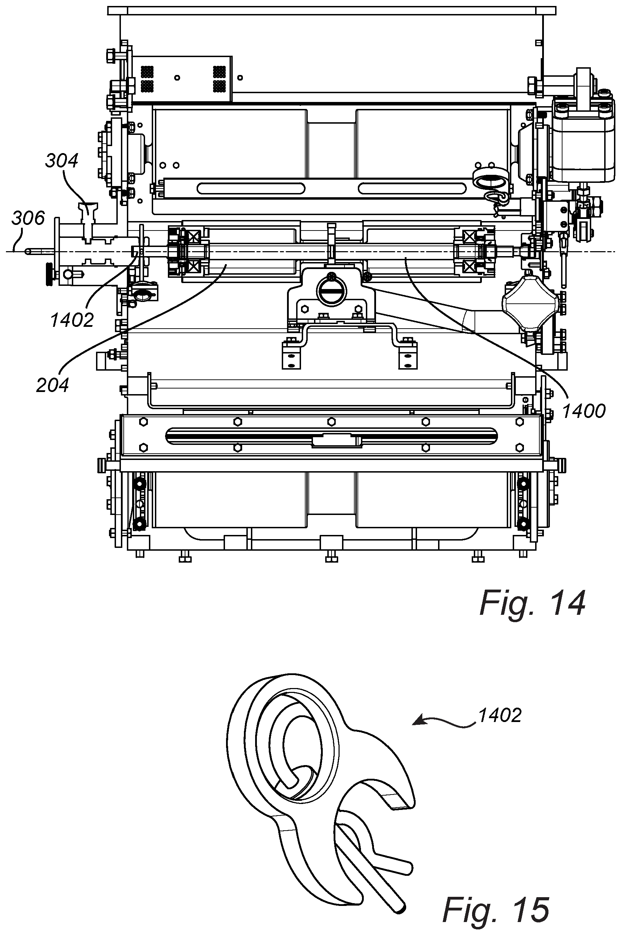

Fig. 14 illustrates a cross-sectional view of the hydrogen peroxide removal station 108 with the CRA 200 removed. As illustrated, a SRA shaft 1400 of the SRA 204 may be secured by using a SRA locking pin 1402. Similar to the CRA, the SRA lifting knob 304 may be arranged to interact with an operational state slot and a service state slot.

Fig. 15 illustrates the SRA locking pin 1402 in further detail. As illustrated, similar to the CRA locking pin 402, the SRA locking pin may be provided with an R-spring and a ring arranged such that the service technician can hold the locking pin with a finger. The ring's shape, dimensions and/or size may prevent it from falling into the peroxide bath.

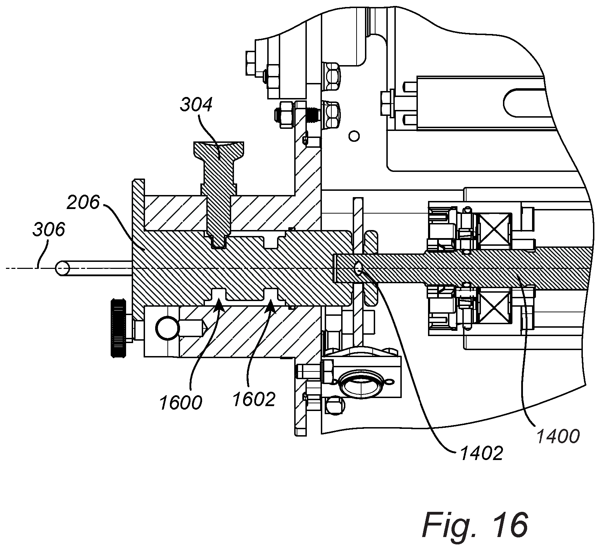

Fig. 16 illustrates the SRA attachment member 206 in further detail. As illustrated and as discussed above, the SRA attachment member may be provided with the operational state slot 1600 as well as the service state slot 1602.

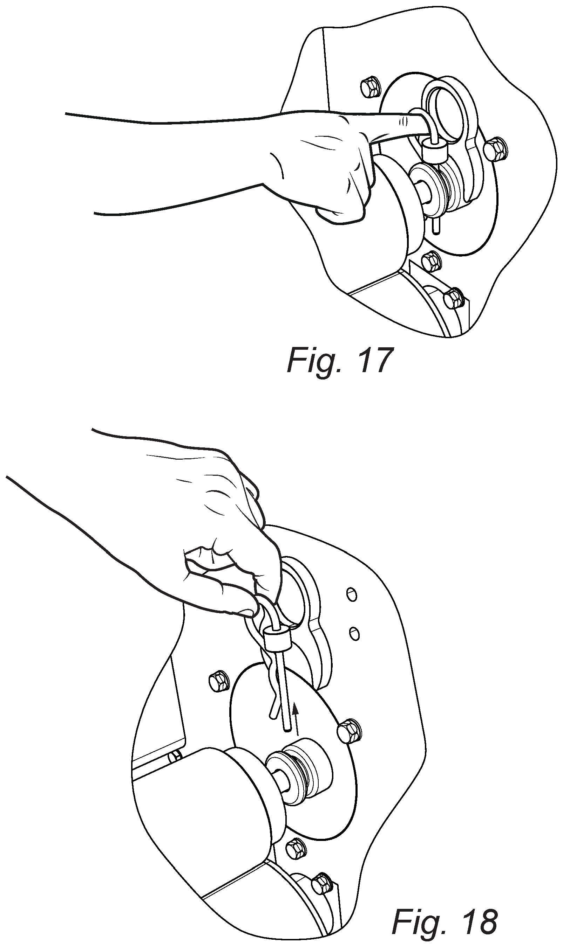

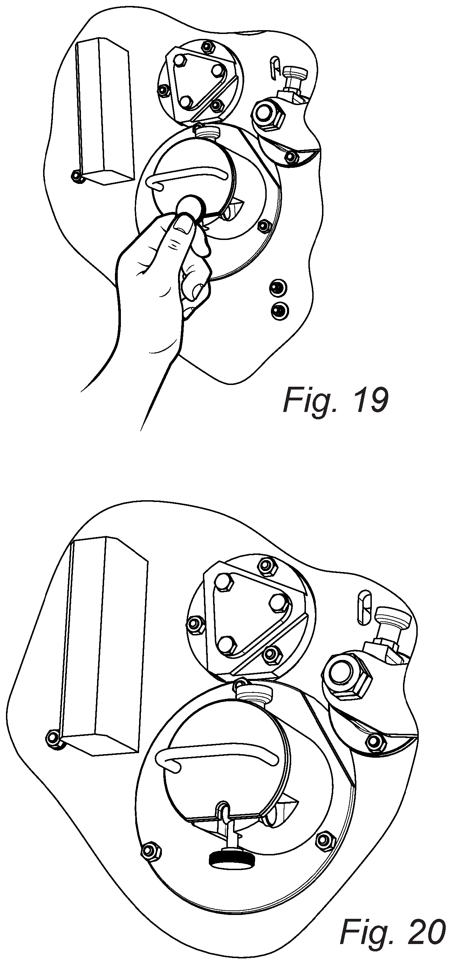

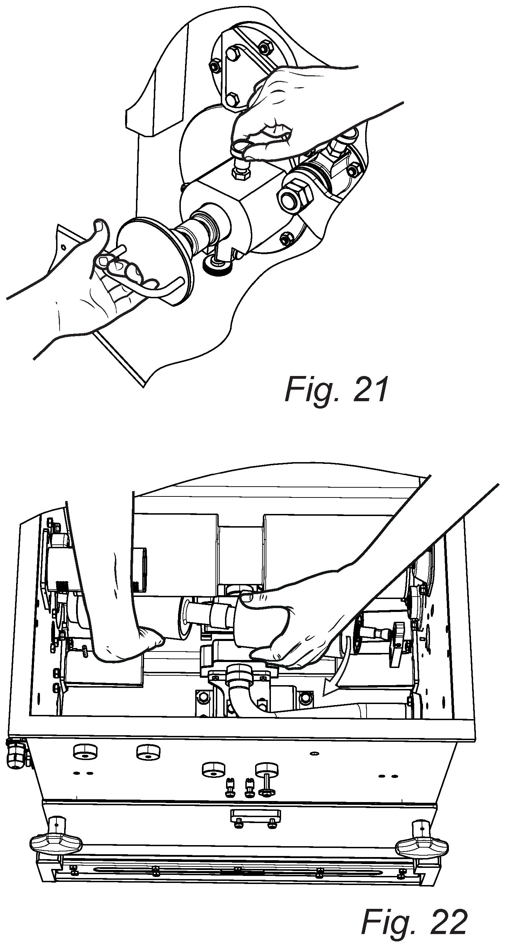

Fig. 17 to 22 illustrate how the SRA 204 can be removed by way of example. Fig. 17 illustrates how the service technician can place his or her finger in the ring of the SRA locking pin. Fig. 18 illustrates the SRA locking pin removed such that the SRA shaft is allowed to slide away from the drying chamber. Fig. 19 illustrates how a pivotally arranged locking member may be tilted downwards by the service technician such that the locking member is placed vertically and pointing downwards, as illustrated in fig. 20. Fig. 21 illustrates how the SRA attachment member 206 is pulled away from the drying chamber by the service technician while lifting the lifting knob and after the locking member has been rotated such that this is pointing downwards. Fig. 22 illustrates how the other end of the SRA, that is, the end being opposite to the end interacting with the SRA attachment member, is released from the drying chamber by rotating the SRA shaft.

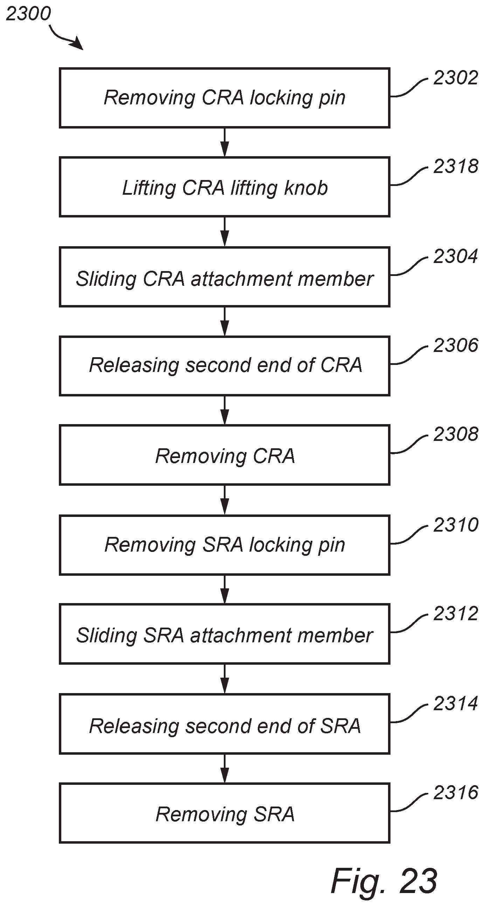

Fig. 23 is a flowchart illustrating a method 2300 for dismounting the hydrogen peroxide removal station 108. The method may comprise removing 2302 the CRA locking pin 402 such that the CRA attachment member 202 is disengaged from the CRA 200, sliding 2304 the CRA attachment member 202 along the CRA pivot axis away from the CRA, releasing 2306 a second end of the CRA 200 by rotating the CRA 200 around the CRA pivot axis, removing 2308 the CRA 200 from the drying chamber 208, removing 2310 the SRA locking pin 1402 such that the SRA attachment member 206 is disengaged from a SRA shaft 1400, sliding 2312 the SRA attachment member 206 along the SRA RA away from the SRA shaft 1400, releasing 2314 a second end of the SRA 204 by rotating the SRA shaft 1400 around the SRA RA, and removing 2316 the SRA 204 from the drying chamber 208. Optionally, the method may further comprise lifting 2318 the CRA lifting knob 300 such that the CRA attachment member 202 is allowed to slide along the CRA pivot axis CRA PA. Further, the method may also comprise lifting the SRA lifting knob 304 such that SRA attachment member is allowed to slide along the SRA shaft 1400.
From the description above follows that, although various embodiments of the invention have been described and shown, the invention is not restricted thereto but may also be embodied in other ways within the scope of the subject-matter defined in the following claims.

### List of references

100 - filling machine / packaging machine
102 - reel of packaging material
104 - web of packaging material
106 - hydrogen peroxide bath
108 - hydrogen peroxide removal station
110 - tube of packaging material
112 - longitudinal sealing LS station
114 - transversal sealing TS station
116a - first chain
116b - second chain
118 - packages
200 - calendar roller assembly CRA
202 - CRA attachment member
204 - squeegee roller assembly SRA
206 - SRA attachment member
208 - drying chamber
300 - CRA lifting knob
302 - CRA rotational axis, CRA RA
304 - SRA lifting knob
306 - SRA rotational axis RA
400 - CRA shaft
402 - CRA locking pin
404 - first CRA side section
406 - second CRA side section
408 - CRA bar
410 - fixed CRA attachment member
412 - hole
500 - R-spring
502 - ring
700 - operational state slot
702 - service state slot
1400 - SRA shaft
1402 - SRA locking pin

## Claims

1. A method (2300) for dismounting a hydrogen peroxide removal station (108) arranged for removing residual hydrogen peroxide from a web (104) of packaging material downstream a hydrogen peroxide bath (106) in a filling machine (100), wherein the hydrogen peroxide removal station (108) comprises a calendar roller assembly (CRA) (200) comprising a CRA shaft (400), a squeegee roller assembly (SRA) (204) comprising a SRA shaft (1400), a drying chamber (208), a CRA attachment member (202) for holding the CRA (200) in position within the drying chamber (208), and a SRA attachment member (206) for holding the SRA (204) in position within the drying chamber (208), said method comprising
removing (2302) a CRA locking pin (402) such that the CRA attachment member (202) is disengaged from the CRA (200),
sliding (2304) the CRA attachment member (202) along a CRA pivot axis away from the CRA,
releasing (2306) a second end of the CRA (200) by rotating the CRA 200 around the CRA pivot axis,
removing (2308) the CRA (200) from the drying chamber (208),
removing (2310) a SRA locking pin (1402) such that the SRA attachment member (206) is disengaged from a SRA shaft (1400),
sliding (2312) the SRA attachment member (206) along the SRA rotational axis away from the SRA shaft (1400),
releasing (2314) a second end of the SRA (204) by rotating the SRA (204) around the SRA RA, and
removing (2316) the SRA (204) from the drying chamber (208).

2. The method according to claim 1, wherein, during operation, hot air is introduced into the hydrogen peroxide removal station (108).

3. The method according to any one of the preceding claims, wherein the drying chamber (208) comprises a first side section and a second side section, wherein the CRA attachment member (202) and the SRA attachment member (206) are arranged in a first and a second opening in the first side section, wherein the CRA locking pin (402) and the SRA locking pin are provided inside the drying chamber (208), and a CRA and a SRA grip are provided outside the drying chamber (208) such that the steps of sliding the CRA attachment member (202) and sliding the SRA attachment member (206) can be achieved by pulling the grips away from the drying chamber (208).

4. The method according to any one of the preceding claims, wherein a CRA lifting knob (300) is provided, said method further comprising
lifting (2318) the CRA lifting knob (300) such that the CRA attachment member (202) is allowed to slide along the CRA pivot axis.

5. The method according to claim 4, wherein the CRA attachment member (202) is provided with an operational state slot (700) and a service state slot (702) such that by lifting the CRA lifting knob (300) and sliding the CRA attachment member (202) along the CRA pivot axis while the CRA lifting knob is lifted, the CRA lifting knob (300) can be moved between the operational state slot (700) and the service state slot (702).

6. The method according to claim 4 or 5, wherein the CRA lifting knob is spring-loaded.

7. The method according to any one of the preceding claims, wherein the CRA (200) further comprises a first CRA side section (404), a second CRA side section (406) and a CRA bar (408), wherein the CRA shaft (400) is connected to a first end of the first and second CRA side section and the CRA bar (408) is connected to a second end of the first and second CRA side section (404, 406).

8. The method according to claim 7, wherein, in an operational state, the CRA attachment member (202) is engaging with the first CRA side section and a fixed CRA attachment member (410) is engaging with the second CRA side section, wherein the fixed CRA attachment member (410) is fed through a hole (412) in the second CRA side section, and the CRA locking pin (402) is attached to the fixed CRA attachment member (410) such that movement of the CRA (200) along the CRA pivot axis is prevented.

9. The method according to any one of the preceding claims, wherein the CRA locking pin (402) comprises an R-spring (500).

10. The method according to claim 8, wherein the CRA locking pin (402) further comprising a ring (502) having a diameter of 2-4 cm, wherein the ring (502) is attached to the R-spring (500).

11. A hydrogen peroxide removal station (108) comprising a calendar roller assembly (CRA) (200) comprising a CRA shaft (400),
a squeegee roller assembly (SRA) (204) comprising a SRA shaft (1400),
a drying chamber (208),
a CRA attachment member (202) for holding the CRA (200) in position within the drying chamber (208), and
a SRA attachment member (206) for holding the SRA (204) in position within the drying chamber (208),
a CRA locking pin (402) arranged to be placed in a hole in the CRA (200) such that the CRA (200) is prevented from moving,
wherein the CRA attachment member (202) is arranged to be slid along a CRA pivot axis away from the CRA after the CRA locking pin (402) is removed,
a SRA locking pin (1402) arranged to be placed in a hole in the SRA (204) such that the SRA r (204) is prevented from moving,
wherein the SRA attachment member (206) is arranged to be slid along a SRA rotational axis (SRA RA) away from the SRA shaft (1400) after the SRA locking pin is removed.

12. The hydrogen peroxide removal station according to claim 11, further comprising
a hot air nozzle arranged, during operation, to feed in hot air into the hydrogen peroxide removal station (108).

13. The hydrogen peroxide removal station according to claim 10 or 11, wherein the drying chamber (208) comprises a first side section and a second side section, wherein the CRA attachment member (202) and the SRA attachment member (206) are arranged in a first and a second opening in the first side section, wherein the CRA locking pin (402) and the SRA locking pin is provided inside the drying chamber (208), and a CRA and a SRA grip are provided outside the drying chamber (208).

14. A filling machine (100) comprising a hydrogen peroxide bath (106) and the hydrogen peroxide removal station (108) according to any one of the claims 11 to 13.

15. A kit of parts for upgrading an existing hydrogen peroxide removal station comprising a drying chamber (208), said kit of parts comprising
a calendar roller assembly (CRA) (200) comprising a CRA shaft (400),
a squeegee roller assembly (SRA) (204) comprising a SRA shaft (1400),
a CRA attachment member (202) for holding the CRA (200) in position within the drying chamber (208), and
a SRA attachment member (206) for holding the SRA (204) in position within the drying chamber (208),
a CRA locking pin (402) arranged to be placed in a hole in the CRA (200) such that the CRA (200) is prevented from moving,
wherein the CRA attachment member (202) is arranged to be slid along a CRA pivot axis away from the CRA after the CRA locking pin (402) is removed,
a SRA locking pin (1402) arranged to be placed in a hole in the SRA (204) such that the SRA (204) is prevented from moving,
wherein the SRA attachment member (206) is arranged to be slid along a SRA rotational axis (SRA RA) away from the SRA shaft (1400) after the SRA locking pin is removed.
